# DEMANDE DE BREVET EUROPEEN

(11) **EP 2 269 509 A1**
(43) Date de publication de la demande: **05.01.2011**
(21) Numéro de dépôt: 09008438.5
(22) Date de dépôt: 29.06.2009
(51) Int. Cl.: A61B 5/15

(54) **Dispositif intégré incorporant un cathéter porte-capillaire et une tige à aiguille pour faciliter le prélèvement securitaire de sang foetal**

(71) Demandeur: Prothia S.A.R.L., 75020 Paris (FR)
(72) Inventeur: Spear, Brian Hartwell, 7513 Paris (FR)

(57) **Abrégé**

Dispositif intégré (représenté dans la figure 1A) incorporant un cathéter (1) qui peut porter un capillaire (13) et une tige (2) munie d'une aiguille (8) pour faciliter et sécuriser le prélèvement de sang foetal, destiné à des mesures biologiques. Il s'agit d'un dispositif composé d'une tige (2), d'une aiguille (8) et d'un cathéter (1) qui peut porter un capillaire (13) et une tige (2) qui se déplace dans la lumière (6) constituée par le cathéter (1) et le capillaire (13). Le dispositif est caractérisé par le fait qu'un seul ensemble intégré sert à faire l'incision, à remplir le capillaire (13) ainsi qu'à introduire et retirer le capillaire (13) dans de bonnes conditions. La lumière interne centrale (6) dans laquelle la tige (2) se déplace constitue une innovation technique. De façon similaire la tige (2) a une construction particulière pour limiter la pénétration de l'aiguille (8), particulièrement la forme de l'extrémité distale et le diamètre réduit (10) sur une partie de la tige (2).

## Description

La présente invention concerne un dispositif pour prélever du sang du foetus, notamment dans le cadre du travail d'accouchement. L'invention peut servir pour d'autres prélèvements que ceux de sang foetal.

De nombreuses techniques médicales servent à évaluer l'état du foetus pendant l'accouchement. L'objectif de ces moyens de mesure et de surveillance est d'évaluer le bien être foetal et son évolution. L'analyse de l'électrocardiogramme du foetus, l'étude de la tendance du rythme cardiaque foetal et de l'oxymétrie du pouls foetal figurent parmi les méthodes utilisées en obstétrique pour suivre l'évolution du foetus et pour guider les réponses thérapeutiques voire chirurgicales à une éventuelle détérioration. Les obstétriciens, sages-femmes et autres cliniciens responsables des accouchements, tentent d'éviter que le foetus ne souffre. Plus particulièrement une oxygénation insuffisante du cerveau due à une perfusion cérébrale inadéquate peut créer des lésions permanentes. Une pathologie qui peut être liée à cette hypoxie foetale est l'infirmité motrice centrale (IMC).

Souvent, les cliniciens souhaitent associer une analyse biologique sanguine aux méthodes de surveillance de signaux physiologiques. Les cliniciens peuvent s'intéresser à différents paramètres biologiques, traditionnellement le paramètre de référence pour évaluer l'état du foetus est l'acidité du sang : le pH. Depuis quelques années, les cliniciens s'intéressent également à la concentration de lactate dans le sang. Ces différentes mesures nécessitent la prise d'un petit échantillon de sang foetal (dans certains cas de seulement quelques micro litres).

Le prélèvement de sang foetal dans de bonnes conditions de sécurité pose de nombreux problèmes. Le clinicien n'a pas un accès direct à la peau comme chez l'adulte et doit réaliser son geste dans l'utérus (in utero). En utilisant des techniques lui permettant de travailler à distance du sujet, il doit trouver le moyen de nettoyer la peau du cuir chevelu du foetus, pratiquer une incision, isoler le sang des liquides environnants, faire entrer le sang dans un compartiment sans que sa nature ne soit trop modifiée, pour ensuite le récupérer et l'introduire dans un dispositif de mesure.

Des dispositifs actuels réalisent parfois des incisions plus importantes que nécessaires et obligent les cliniciens à faire des gestes précis, difficiles à réaliser surtout dans les conditions intra-utérines souvent à l'aide d'un amnioscope et au cours d'un travail d'accouchement.

La présente invention se propose d'apporter une aide à la réalisation du prélèvement du sang foetal et tient compte des difficultés et défis rencontrés par les cliniciens et évoqués ci-dessus. En même temps elle vise à réduire les risques liés aux prises de sang in utero. Le dispositif comprend une tige à aiguille conçue pour réaliser une incision moins traumatique et un cathéter porte capillaire pour recueillir l'échantillon sanguin et en faciliter le transport.

Au cours des dernières décennies il y a eu des inventions qui traitent des problèmes similaires à ceux proclamés par la présente invention. En 1984 Monsieur Philippe Jeanty a breveté une invention pour faciliter les prélèvements sur le cordon et non pas l'accès au cuir chevelu foetale.

Monsieur Michaël Worley (1984) a inventé un système d'aspiration par aiguille ne permettant pas de limiter la force utilisée pour l'incision; et les risques de trauma associées.

Monsieur Edward Adamowicz a inventé un système de prélèvement de sang foetale basé sur un principe de coupe en biais avec une lame sans moyen de limiter les risques d'une incision trop large et trop profonde.

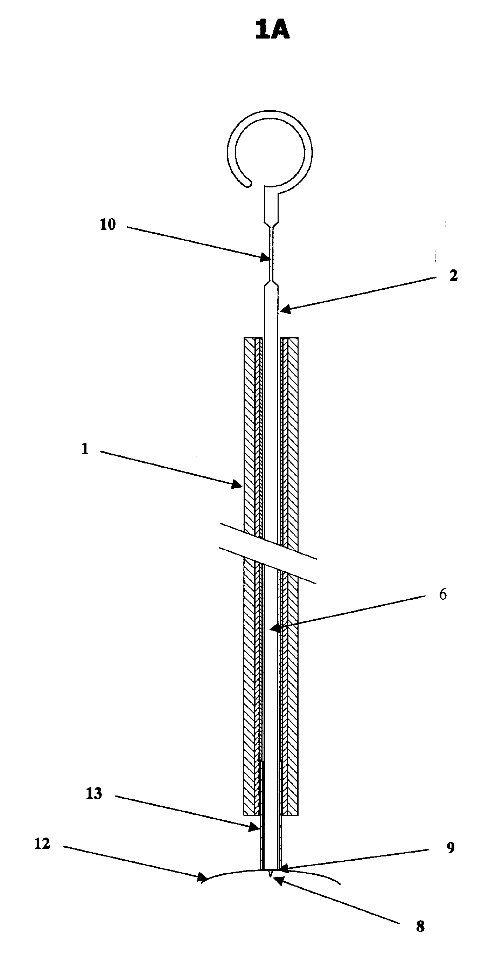

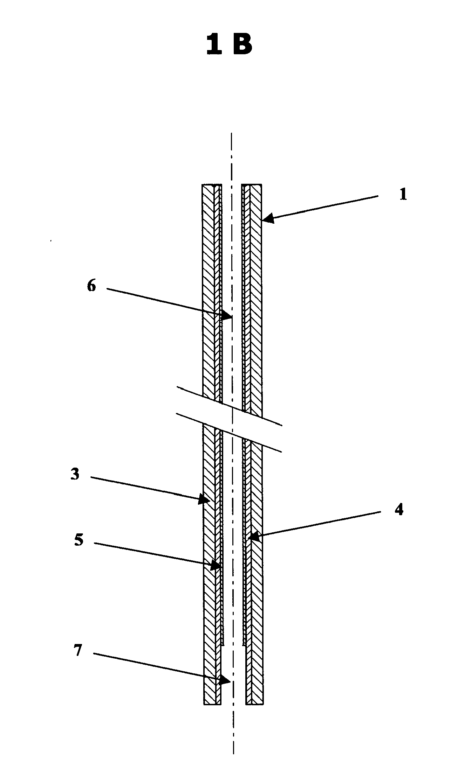

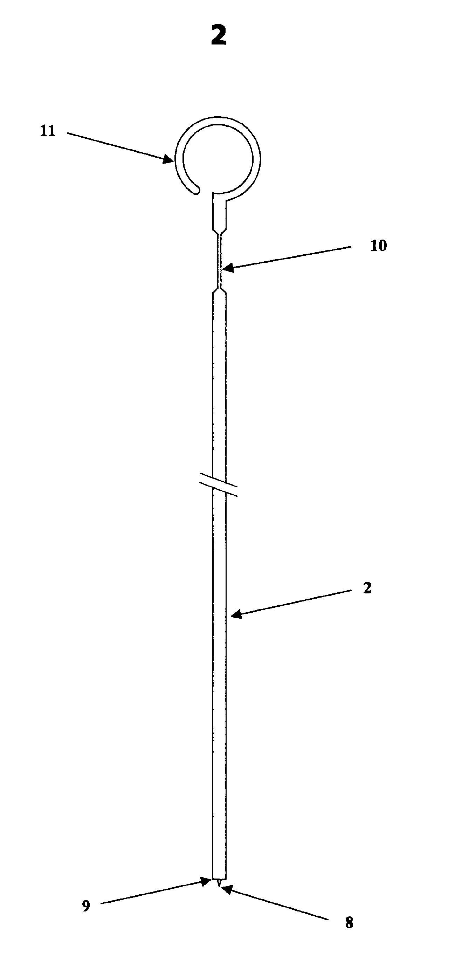

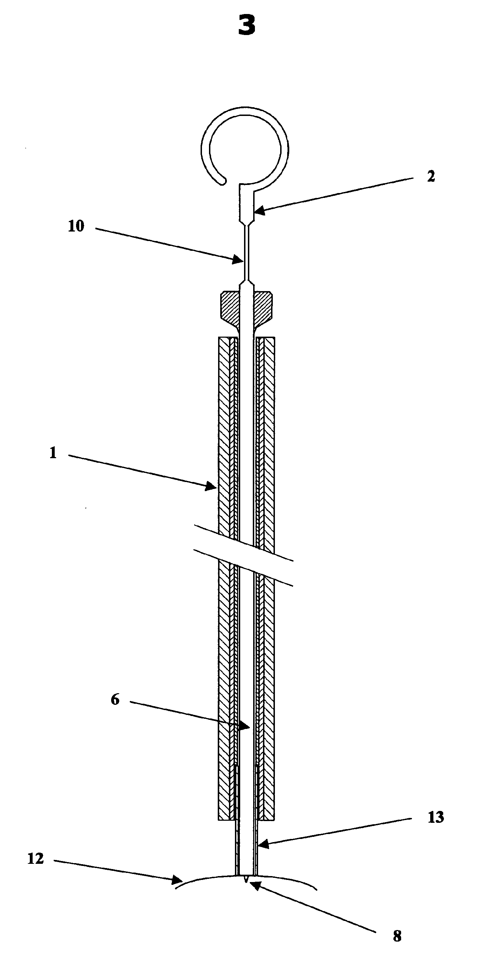

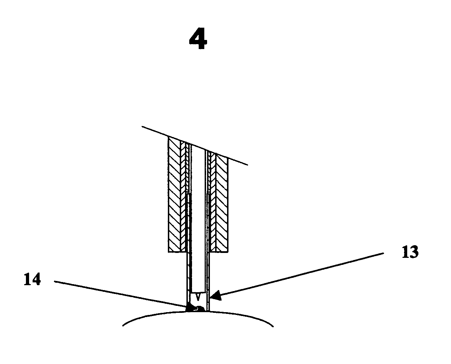

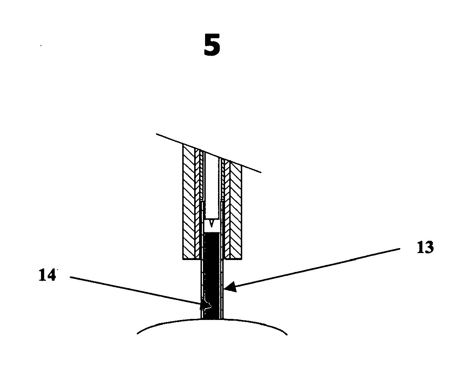

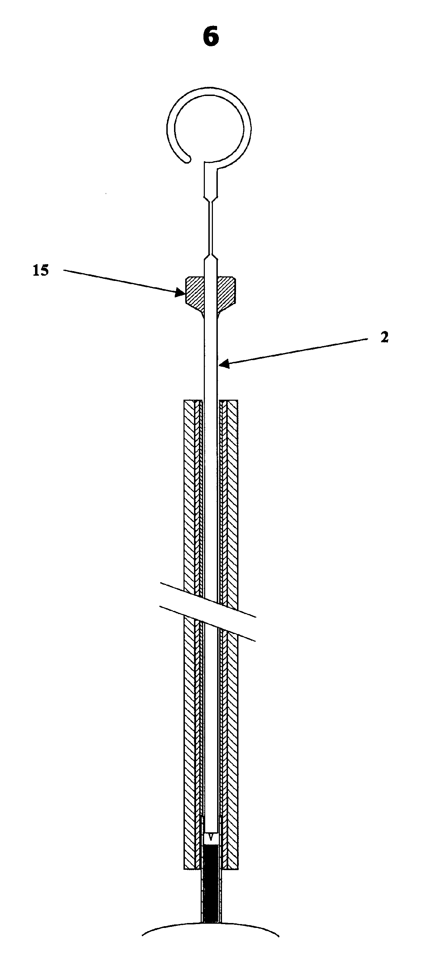

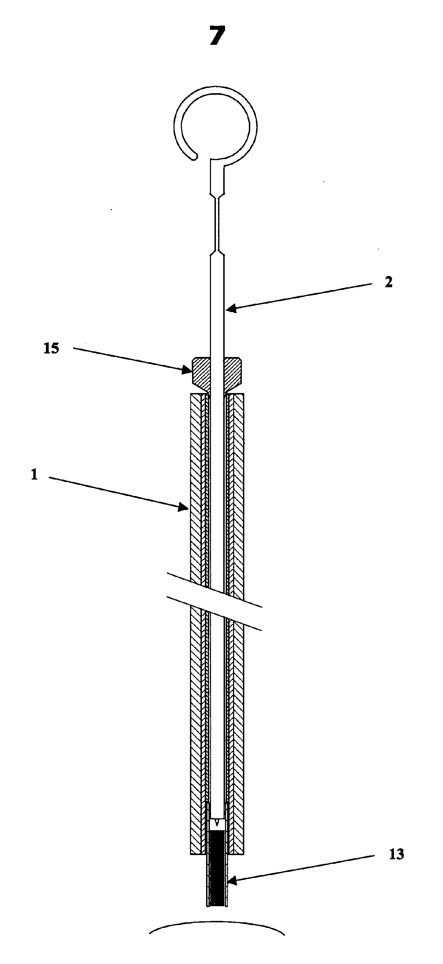

On décrira à présent l'invention en référence aux dessins annexés dans lesquels la figure 1A représente l'invention dans son ensemble, la figure 1B représente le cathéter (1), la figure 2 la tige à aiguille (2), la figure 3 la tige à aiguille (2) introduite au fond de l'ensemble [cathéter porteur (1) + capillaire (13)] avec l'aiguille (8) en cours de ponction, la figure 4 la première goutte de sang foetal (14) à l'intérieur du capillaire (13), la figure 5 le capillaire (13) rempli de sang foetal (14), la figure 6 le verrou (15) de la tige en position d'ouverture, la figure 7 le même verrou (15) en position de fermeture. Il est proposé de publier avec l'abrégé la figure 1BA.

Dans la figure 1B, on voit la cavité (7) au niveau de l'embout distal du cathéter (1) destinée au capillaire. Selon un aspect avantageux de l'invention, bien que non limitatif, la paroi de la cavité est légèrement adhérente pour assurer la rétention du capillaire. Le capillaire lors de son introduction élargit la cavité dont la plasticité est assurée par les matériaux utilisés. La fermeture de la cavité sur le capillaire associée à la qualité adhérente de la paroi maintient le capillaire en place dans le cathéter qui dispose d'une lumière interne (6). Le cathéter est composé de 2 couches rigides : la couche extérieure (3) et la couche intérieur(5), ainsi qu'une couche souple et légèrement adhérente : la couche intermédiaire (4).

La figure 2 représente la tige à aiguille (2). L'extrémité proximale de la tige est munie d'une boucle (11). L'extrémité distale est munie d'une aiguille (8). Selon un aspect avantageux de l'invention, bien que non limitatif, l'aiguille (8) se trouve au centre de cette extrémité munie d'un aplat (9) de sorte que la circonférence de la tige butte contre la peau du foetus et limite la profondeur de pénétration. Une partie (10) de la tige a un diamètre réduit pour plier sous une force trop élevée, ceci pour limiter le risque de faire pénétrer trop profondément la tige.
Dans la figure 3, on voit la pénétration de l'aiguille (8) dans la peau (12) (cuir chevelu). La force de pénétration est fournie par le doigt du clinicien et limitée par la partie de la tige à diamètre réduit (10). On voit que la tige (2) se déplace dans la lumière (6) créée par le cathéter (1) et le capillaire (13).

La figure 4 montre la première goutte de sang foetal (14) à l'intérieur du capillaire (13).

La figure 5 montre le capillaire (13) rempli de sang foetal (14). Selon un aspect avantageux de l'invention, bien que non limitatif, la tige (2) fonctionne tel piston et crée un léger vide, lequel associé à l'effet de capillarité favorise la montée du sang (14) dans le capillaire (13).

Dans la figure 6 on voit le verrou (15) en position d'ouverture. Dans cette position la tige (2) avance et recule sans frein. L'obturateur glisse sur la tige sous une force modérée des doigts du clinicien.

Dans la figure 7 on voit le verrou (15) en position de fermeture. Dans cette position la tige (2) se trouve bloquée. Aussi le clinicien peut transporter le sang sans risque de coulage grâce au maintien de la tige à la fermeture et à l'entrée d'air dans le cathéter (1). Pour faire couler le sang, il suffit soit de séparer le capillaire (13) et le cathéter (1) soit de retirer ensemble la tige (2) et le verrou (15).

## Revendications

1. Dispositif de prélèvement de sang foetal, **caractérisé en ce qu'**un cathéter (1) est formé de trois couches plastiques concentriques dont une rigide extérieure (3), une rigide intérieure (5) et une souple intermédiaire (4) de sorte que cette dernière retienne le capillaire (13) dans une cavité (7).

2. Dispositif selon la revendication 1 **caractérisé en ce qu'**une lumière interne centrale (6) comprend le capillaire (13) et le cathéter pour placement in utero sur le crâne du foetus et dont l'extrémité proximale se trouve au-delà du col ex utero de telle sorte qu'une tige (2) munie d'une pointe en aiguille (8) se déplace d'une extrémité à l'autre de la lumière (6) et fonctionne comme un piston de manière à faciliter l'entrée et la sortie de sang foetal (14) dans le capillaire (13).

3. Dispositif selon une quelconque des revendications 1 ou 2 **caractérisé en ce que** la tige (2), pour limiter la force transmise à l'aiguille (8), a un diamètre réduit sur la partie proximale (10) par rapport à l'extrémité proximale du cathéter (1).

4. Dispositif selon une quelconque des revendications précédentes **caractérisé en ce que** l'extrémité proximale de la tige (2) forme une boucle (11) pour recevoir le doigt du clinicien afin de faciliter l'avancement et le recul de la tige (2) à l'intérieur de la lumière centrale (6).

5. Dispositif selon une quelconque des revendications précédentes **caractérisé en ce qu'**il comprend un verrou proximal mobile (15) afin de maintenir en place la tige pour éviter que le sang ne coule en dehors du capillaire (13).
